# EUROPEAN PATENT SPECIFICATION

(11) **EP 1 069 130 B1**
(45) Date of publication and mention of the grant of the patent: **11.08.2004**
(21) Application number: 99401772.1
(22) Date of filing: 15.07.1999
(51) Int. Cl.: C07H 13/12, A61K 31/70

(54) **Moenomycin A Derivatives, their preparation, and use as antibacterial products**
Moenomycin-A-Derivate, ihre Herstellung, und ihre Verwendung als Antibakterielle-Arzneimittel
Dérivés de Moenomycin A, leur préparation, et leur utilisation comme produits antibactériens

(43) Date of publication of application: 17.01.2001
(73) Proprietor: HOECHST MARION ROUSSEL, 92800 Puteaux (FR)
(72) Inventor: Lampilas, Maxime, 93230 Romainville (FR); Vogel, Stefan, 04103 Leipzig (DE)
(74) Representative: Vieillefosse, Jean-Claude

(56) References cited:
- EP-A- 0 355 679
- EP-A- 0 652 205
- EP-A- 0 655 249
- WO-A-99/26956
- KEMPIN U ET AL: "Moenomycin A: New Chemistry that Allows to Attach the Antibiotic to Reporter Groups, Solid Supports, and Proteins" TETRAHEDRON,NL,ELSEVIER SCIENCE PUBLISHERS, AMSTERDAM, vol. 53, no. 52, page 17669-17690 XP004126736 ISSN: 0040-4020

## Description

The invention relates to new derivatives of moenomycin A, their process of preparation, and their use as antibacterial products.

The invention relates to compounds of formula (1) 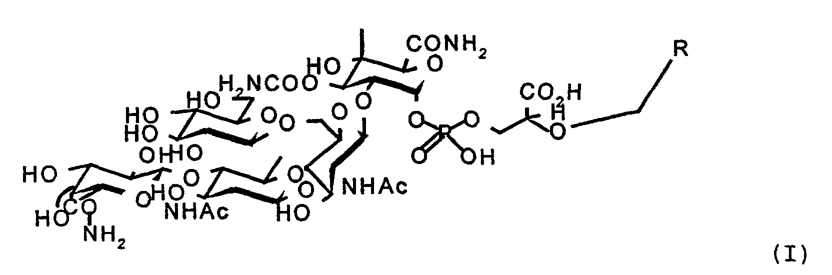 wherein
R is a radical 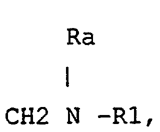 Ra being an hydrogen atom or an alkyl radical up to 8 carbon atoms, R1 being an hydrogen atom, an optionally substituted alkyl radical linear or branched up to 30 carbon atoms, saturated or unsaturated or an optionally substituted aryl radical up to 14 carbon atoms, or R is a radical 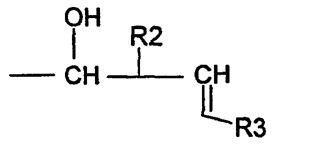 R2 being an hydrogen atom or an alkyl radical linear or branched saturated or unsaturated up to 30 carbonatoms, R3 being an hydrogen atom or an alkyl radical up to 12 carbon atoms. The aryl is preferably phenyl or naphtyl. Examples of alkyl, alkenyl or alkynyl are preferably methyl, ethyl, propyl, isopropyl, n-butyl, isobutyl, terbutyl, octyl, decyl, dodecyl, tetradecyl, pentadecyl, hexadecyl, octadecyl, vinyl, ethynyl, propynyl, propargyl, cyclobutyl, cyclopentyl or cyclohexyl.

Among the preferred compounds of formula I are those wherein R is a radical 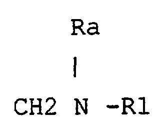 Ra and R1 being defined as above for exemple those wherein Ra is an hydrogen atom and those wherein R1 is an aryl radical substituted by an alkyl radical in position para or a radical

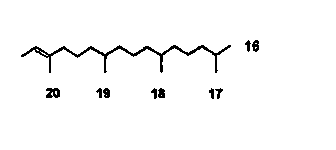

Among the preferred compounds of formula I are also those wherein R is a radical 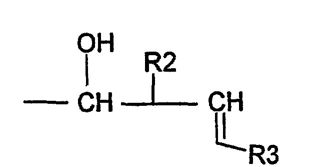 R2 and R3 being defined as above, for example compounds, wherein R2 is an alkyl radical comprising between 7 and 18 carbon atoms.

Among the preferred compounds of formula are compounds the preparation of which is described hereunter and more specially products of examples 4, 5 and 6.
The antibiotic compositions of the invention are comprised of an antibiotically effective amount of a compound of formula I and its non-toxic, pharmaceutically acceptable acid addition salts and an inert pharmaceutical carrier. The compositions may be in the form of tablets, dragees, capsules, granules, suppositories, ointments, creams, gels and injectable solutions or suspensions.

Examples of suitable pharmaceutical carriers or excipients are talc, gum arabic, lactose, starch, magnesium stearate, cocoa butter, aqueous or non aqueous vehicles, fatty substances of animal or vegetable origin, paraffin derivatives, glycols, various wetting, dispersing or emulsifying agents and preservatives.

There compositions can also be presented in the form of a powder intended to be dissolved extemporaneously in a suitable vehicle, for example apyrogenic sterile water.

The compositions possess a very good antibiotic activity on gram⊕ bacteria such as S.aureus, S. pyogenes, or E. Faecium. They are useful in the treatment of infections caused by theses germs particularly preferred are compositions using the compounds of Examples 4, 5 and 6.

The novel method of the invention for treating bacterial infections in warm-blooded animals, including humans, comprises, administering to warm-blooded animals an antibiotically effective amount of a compound of formula I or its non-toxic, pharmaceutically acceptable acid addition salts. The compounds may be administered buccally, rectally, parenterally or topically by application to the skin or mucous membrane. The usual daily dose is 1 mg to 1000 mg depending on the condition treated, the specific compound administered and the method of administration.
The process of the invention for the preparation of compounds of formula I comprises reacting moenomycin A to an ozonolysis reaction ,obtaining the compound of formula II 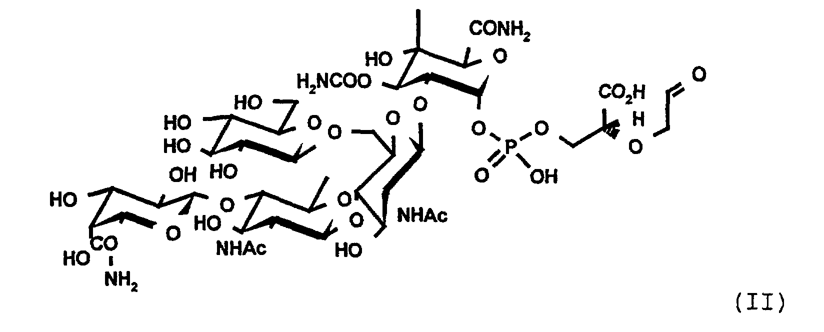 then submitting the compound of formula II to the action of a reagent or several reagents capable of remplacing 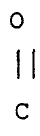 by the radical CHR, and obtaining the corresponding compound of formula I.

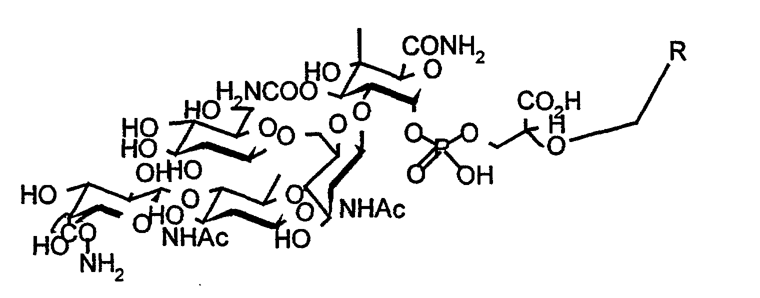

For example, the invention relates to a process wherein the compound of formula II is reacted with an amine of formula III.

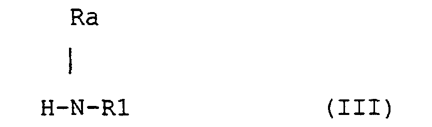

As a reduction agent, NaBH₃CN may be used.
As an ambodiment of the invention there is a process wherein a compound of formula II is submitted to an allylhalogenide in presence of indium powder to get the compound of formula I wherein R is a radical 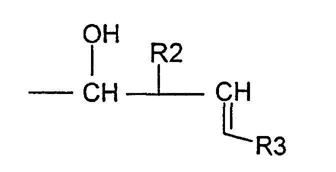 wherein R is a radical R2 and R3 being defined as above. The compound of formula II is an objet of this invention. The invention relates also to compounds of formula. I as feed additives. Another objet of the invention is feed additives including as antibacterial products at least one compound of formula I..

The feed additives are preferably administered to the animals orally in the form of tablets, granules or powders incorporated into the feed for pigs or calves prepared by the known procedures of such products but they may also be administered parenterally.
The feed mixture will vary depending on the animal species but usually contains cereals, sugars, grains, arachidic and tournsole and soybean press cake, flours of animal origin such as fish flour, amino acids of synthesis, mineral salts, vitamins and antioxidants.
In the following examples, there are described several preferred embodiments to illustrate the invention. However, it is to be understood that the invention is not intended to be limited to the specific embodiments.
In the NMR spectra the proton and carbon are assigned according to the following convention.

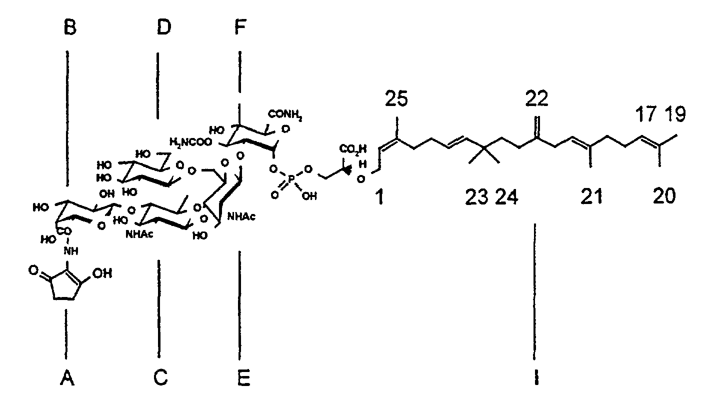

### Example 1 : Hexadecylamine derivative

### Step A

### Synthesis of the aldehyde of moenomycin A (P)

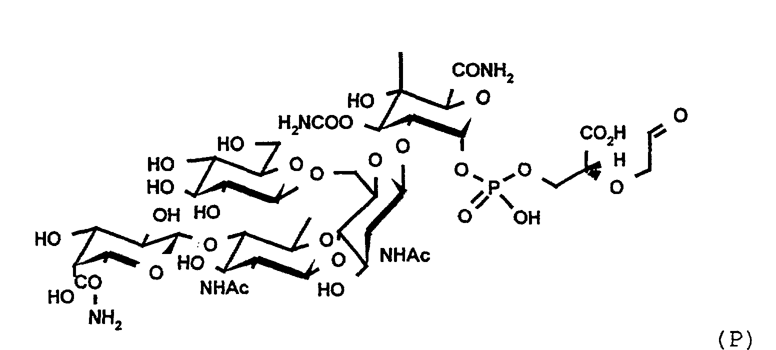

A solution of moenomycin A (1061.7 mg, 671 pmol) in 15 ml of methanol was cooled to -78°C. The solution was saturated with ozonised oxygen (Fischer OZON 502, flow rate 50 l/h = 2 g/h O₃) at -78 °C until the solution reach a light blue colour (ca. 30 min). The precipitated product was filtered after warming up to room temperature and rinsed with methanol. The product was solved in water and lyophilised to give the expected product in 98 % (786.4 mg) yield.
C₄₁H₆₆N₅O₃₃P1187.9 g/mol
MS: m/z (electron spray - negative mode)
1186 ([M-H]⁻), 1144 ([M-H-CH₂CO]⁻), 592 ([M-2H]²⁻), 571 ([M-2H-CH₂CO]²⁻)
¹H-NMR δ = 1.20 (s, CH₃-4^{F}), 1.28 (d, CH₃-6^{C}), 2.00 (s,NHCOCH₃^{E}), 2.07 (s,NHCOCH₃^{C}), 4.44 (s, H-5^{F}), 4.48 (d, H-1^{C}), 5.68 (q, H-1^{F})

### Step B

### hexadecylamine derivative of the aldehyde (P)

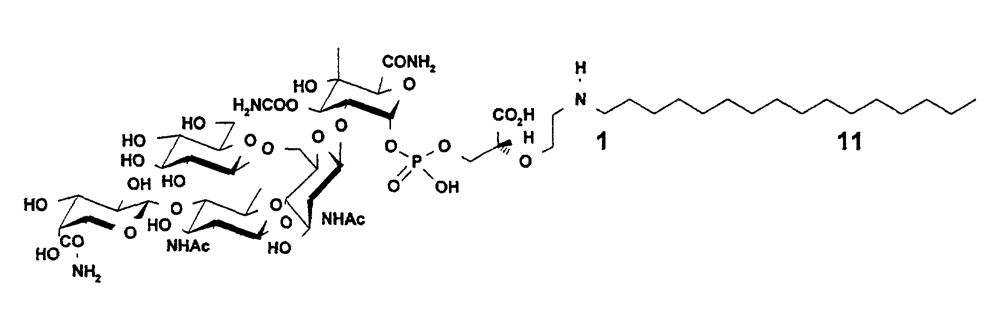

To a solution of **3** (55.6 mg, 47 µmol) in a mixture of 300 µl water, 100 µl phosphate buffer (pH 7, 0.2 M) and 800 µl methanol 46.1 mg (94 µmol) of 1-hexadecylamine solved in 800 µl methanol and 6 mg (94 µmol) NaBH₃CN solved in 100 µl methanol were added. The pH of the mixture was adjusted with 1 N acetic acid to 6.5. The mixture was stirred for 3 days at room temperature. The crude mixture was directly applied on a Sephadex® LH-20 column (10 g/ H₂O-CH₃OH 1 :4). All fractions containing the product were collected, evaporated and freeze dried. The crude product was purified on an silica gel column using AcOEt/ i-PrOH/H₂O (6 :4 :3) as eluent followed by another Sephadex LH-20 column to remove traces of silica gel. The residue was taken up and freed from a second equivalent of 1-hexadecylamine using a DOWEX® (type : 50 W X8) column (water). After solvent evaporation and lyophilisation the product **10** was obtained in 63 % (42.1 mg) yield.
C₅₇H₁₀₁N₆O₃₂P 1413.44 g/mol
MS: m/z (electron spray - positive mode)
1414.3 ([M+H]⁺), 1436.3.9 ([M+Na]⁺), 729.7 ([M+2Na]²⁺), 707.9 ([M+2H]²⁺)
¹H-NMR (600 MHz, D₂O): δ = 0.76 (t, CH₃-16^{I}), 1.12 (s, CH₃-4^{F}), 1.16-1.28 (m, CH₂-3^{I} -15^{I}), 1.29 (d, CH₃-6^{C}), 1.62 (dd, CH₂-2^{I}), 1.93 (s, NHAc^{C}), 1.99 (s, NHAc^{E}), 2.98 (t, CH₂-I¹), 3.20 (t, 2-H^{D}), 3.84 (d, 6-Hₓ^{D}), 4.12 (s, 5-H^{F}), 4.41 (d, 1-H^{D}), 4.49 (d, 2-H^{C}), 4.92 (d, 3-H^{F}), 5.71 (q, 1-H^{F})
¹³C-NMR (150 MHz, D₂O) : δ = 14.4 (C-16^{I}), 15.1 (CH₃-4^{F}), 17.1 (CH₃-C⁶), 22.7 (NHCOCH₃^{E}), 22.8 (NHCOCH₃^{C}), 23.1 (CH₂-15^{I}), 27.0 (CH₂-I²), 29.6-30.4 (CH₂-3^{I}-13^{I}), 32.4 (CH₂-14^{I}), 42.7 (CH₂-1^{I}), 42.8 (CH₂-NH^{I}), 55.8 (C-2^{E}), 55.9 (C-2^{C}), 61.2 (C-6^{D}), 69.3 (C-6^{E}), 70.2 (C-4^{B}), 71.0 (C-4^{D}), 71.5 (C-2^{B}), 72.5 (C-5^{C}), 72.6 (C-3^{E}), 72.8 (C-3^{C}), 73.2 (C-5^{F}), 73.5 (C-4^{F}), 73.6 (C-2^{D}), 73.9 (C-5^{B}), 74.6 (C-3^{F}), 75.3 (C-3^{D}), 76.2 (C-5^{D}), 76.4 (C-2^{F}), 80.2 (C-4^{E}), 83.5 (C-4^{C}), 94.7 (C-1^{F}), 101.7 (C-1^{C}), 102.4 (C-1^{E}), 103.0 (C-1^{B}), 103.7 (C-1^{D}), 158.6 (C-3^{F}), 173.1 (C-5^{B}), 173.6 (C-5^{C}), 174.9 (C-2^{E}), 175.0 (C-2^{C})

### Example 2

### Synthesis of the allyl derivative of the aldehyde (P)

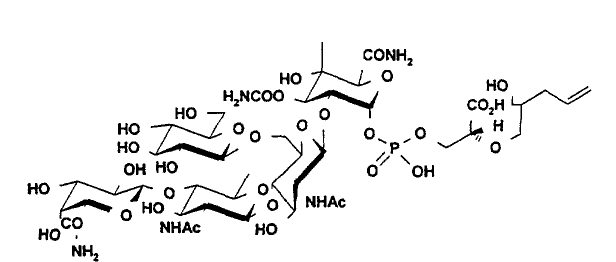

To a solution of product P (21.3 mg, 18 µmol) in a mixture of 600 µl of water and 400 µl methanol allylbromide (4.3 mg, 36 µmol) and indium (4.1 mg, 36 µmol) were added. The mixture was stirred rapidly for 12 h at room temperature. The solvents were removed under reduced pressure from the crude mixture and the residue was resolved in water, after filtration (MILLEX-LCR₁₃®) the filtrate was purified on a Sephadex® PD-10 column. The expected product was obtained in 80.2 % (18.2 mg) yield after lyophilisation.
C₄₄H₇₂N₅O₃₃P - 1230.1 g/mol
¹H-NMR δ = 1.24 (s, CH₃-4^{F}), 1.38 (d, CH₃-6^{C}) , 2.03 (s,NHCOCH₃^{E}), 2.09 (s,NHCOCH₃^{C}) , 4.44 (s, H-5^{F}), 4.57 (d, H-1^{C}), 4.59 (d, H-1^{E}), 5.00 (d, H-3^{F}), 5.85-5.95 (m, -C**H**=CH₂), 5.79 (q, H-1^{F})

### Example 3

### Synthesis of the ammonia derivative of the aldehyde (P)

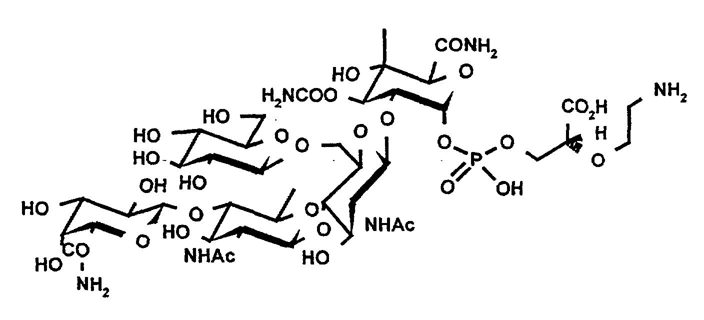

To a solution of product P (99.7 mg, 83 µmol) in an mixture of 400 µl of water and 1600 µl of methanol a solution of ammonium acetate (64.7 mg, 830 µl) and NaBH₃CN (5.35 mg, 160 µmol) in 400 µl of methanol were and the solution stirred for 48 h at room temperature. The crude mixture was applied to a Sephadex LH-20 column to give the expected product **30** in 78 % (77.8 mg) yield.
C₄₁H₆₉N₆O₃₂P1188.99 g/mol
MS: m/z (electron spray - negative mode)
1187.0 ([M-H]⁻), 593.3 ([M-2H]²⁻)
¹H-NMR (200 MHz, D₂O): δ = 1.20 (s, CH₃-4^{F}), 1.28 (d, CH₃-6^{C}), 2.00 (s,NHCOCH₃^{E}), 2.07 (s,NHCOCH₃^{C}), 4.19 (s, H-5^{B}), 4.95 (d, H-3^{F}), 5.8 (q, H-1^{F})
¹³C-NMR (150 MHz, D₂O) : δ = 15.2 (CH₃-4^{F}), 17.1 (CH₃-C⁶), 22.8 (NHCOCH₃^{E}), 22.9 (NHCO CH₃^{C}) , 39.8 (CH₂-NH^{I}), 55.8 (C-2^{E}), 55.9 (C-2^{C}), 61.2 (C-6^{D}), 69.3 (C-6^{E}), 70.1 (C-4^{B}), 71.0 (C-4^{D}), 71.5 (C-2^{B}), 72.4 (C-5^{C}), 72.6 (C-3^{E}), 72.9 (C-3^{C}), 73.1 (C-3^{B}), 73.3 (C-5^{F}), 73.5 (C-4^{F}), 73.6 (C-2^{D}), 73.9 (C-5^{B}), 74.5 (C-3^{F}), 75.2 (C-3^{D}), 76.2 (C-5^{D}), 76.4 (C-2^{F}), 80.1 (C-4^{E}), 83.5 (C-4^{C}), 94.8 (C-1^{F}), 101.6 (C-1^{C}), 102.6 (C-1^{E}), 103.0 (C-1^{B}), 103.6 (C-1^{D}), 158.6 (C-3^{F}), 173.1 (C-5^{B}), 173.6 (C-5^{C}), 174.7 (C-2^{E}), 174.9 (C-2^{C})

### Example 4 : 3-carbamate 1-[(2R)-2-carboxy-2-[[(3,7,11,15-tetramethyl-2-hexadecenyl)amino]ethoxy]ethyl hydrogen phosphate] 0-.beta.-D-glucopyranosyl-(1.fwdarw.6)-O-[O-phosphate] O-.beta.-D-glucopyranosyl-(1.fwdarw.6)-O-[O-(.beta.-D-galactopyranuronamidosyl)-(1.fwdarw.4)-2-(acetylamino)-2,6-dideoxy-.beta.-D-glucopyranosyl-(1.fwdarw.4]-O-2-(acetylamino)-2-deoxy-.beat.-D-glucopyranosyl-(1.fwdarw.2)-4-C-methyl-.alpha.-D-Glucopyranuronamide

### Synthesis of the [¹⁵N]-E-phytolamine derivative of the aldehyde (P)

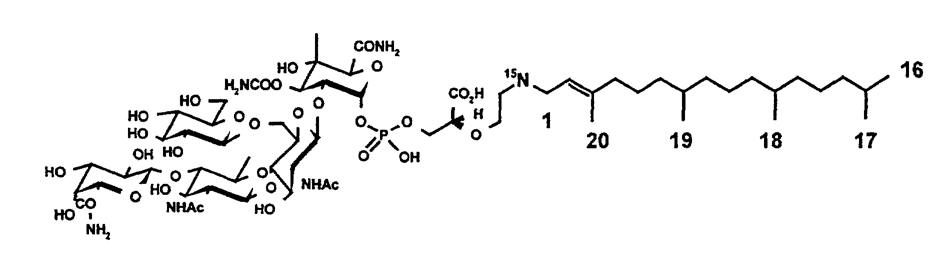

To a solution of product P (54.3 mg, 46 µmol) in a mixture of 300 µl water, 100 µl phosphate buffer (pH 7, 0.2 M) and 800 µl methanol 27.1 mg (92 µmol) of [¹⁵N]-E-phytolamine solved in 800 µl methanol and 5.7 mg (92 µmol) NaBH₃CN solved in 100 µ l methanol were added. The pH of the mixture was adjusted with 1 N acetic acid to 7. The mixture was stirred for 48 h at room temperature. The crude mixture was directly applied on a Sephadex® LH-20 column (10 g/ H₂O-CH₃OH 1 :4). All fractions containing the product were collected, evaporated and freeze dried. The crude product was purified on an silica gel column using AcOEt/ i-PrOH/H₂O (6 :4 :3) as eluent followed by another Sephadex LH-20 column to remove traces of silica gel. The residue was taken up and freed from a second equivalent of [¹⁵N]-E-phytolamine using a DOWEX® (type : 50 W X8) column (water). After solvent evaporation and lyophilisation the product **14** was obtained in 19 % ( 13.2 mg) yield.
C₆₁H₁₀₇N₆O₃₂P 1468.53 gl/mol
MS: m/z (electron spray - positive mode)
1508.6 ([M+K]⁺), 1489.4 ([M+Na]⁺), 754.1 ([M+2K]²⁺)
¹H-NMR (600 MHz, CD₃OD): δ = 0.80-0.82 (s, CH₃- 16,17^{I}), 0.83 (s, CH₃- 18,19^{I}), 1.17 (s, CH₃-4^{F}), 1.3 (CH₂-I), 1.35 (d, CH₃-6^{C}) , 1.71 (s, CH₃-20^{I}) 1.94 (s,NHCOCH₃^{E}), 2.00 (s,NHCOCH₃^{C}), 2.03 (t, C**H**₂-CH=CH-), 5.03 (d, 3-H^{F}), 5.35 (-C**H**=C**H**^{**I**}), 5.96 (q, H-1^{F})

### Example 5 : 3-carbamate 1-[(2R)-2-carboxy-2-[[(4-dodecylphenyl))amino]ethoxy]ethyl hydrogen phosphate] 0-.beta.-D-glucopyranosyl-(1.fwdarw.6)-O-[O-(.beta.-D-galactopyranuronamidosyl)-(1.fwdarw.4)-2-(acetylamino)-2,6-dideoxy-.beta.-D-glucopyranosyl-(1.fwdarw.4]-O-2-(acetylamino)-2-deoxy-.beat.-D-glucopyranosyl-(1.fwdarw.2)-4-C-methyl-.alpha.-D-Glucopyranuronamide

### Synthesis of the dodecylaniline derivative of the aldehyde (P)

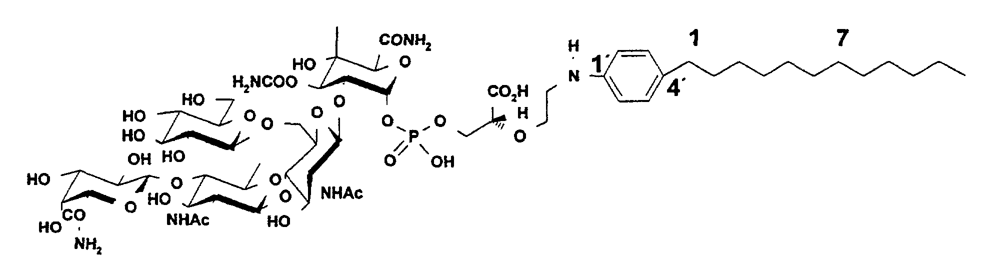

To a solution of product P (53.4 mg, 45 µmol) in a mixture of 300 µl water, 100 µl phosphate buffer (pH 7, 0.2 M) and 800 µl methanol 23.5 mg (90 µmol) of dodecylaniline solved in 800 µl methanol and 5.7 mg (90 µmol) NaBH₃CN solved in 100 µl methanol were added. The pH of the mixture was adjusted with 1 N sodium hydroxide to 7. The mixture was stirred for 72 h at room temperature. The crude mixture was directly applied on a Sephadex® LH-20 column (10 g/ H₂O-CH₃OH 1 :4). All fractions containing the product were collected, evaporated and freeze dried. The crude product was purified on an silica gel column using AcOEt/ i-PrOH/H₂O (6 :4 :3) as eluent followed by another Sephadex LH-20 column to remove traces of silica gel. After solvent evaporation and lyophilisation the desired product was obtained in 31 % (20.1 mg) yield.
C₅₉H₉₇N₆O₃₂P1433.43 g/mol
Ms : M/Z (Maldi-tof) M+=1432.65
¹H-NMR (600 MHz, D₂O): δ = 0.77 (t, CH₃ -12^{I} ), 1.13 (s, CH₃-4^{F}), 1.15-1.22 (CH₂-3^{I}-11^{I}), 1.28 (d, CH₃-6^{C}), 1.49 (CH₂-2^{I}), 1.95 (s, NHCOCH₃^{E}) , 2.01 (s, NHCOCH₃^{C}), 2.45 (t, CH₂-1^{I}), 3.22 (2-H^{D}), 3.66 (6-H_{y}^{D}), 3.77 (*J*=9.9 Hz, 2-H^{F}), 3.83 (*J*=12.5 Hz, 6-Hₓ^{D}), 4.37 (s, 5-H^{F}), 4.43 (d, 1-H^{D}), 4.50 (d, 2-H^{C}), 4.96 (d, 3-H^{F}), 5.71 (q, H-1^{F}), 6.78 (2-H^{Ar}, 6-H^{Ar}), 7.07 (3-H^{Ar}, 5-H^{Ar})
¹³C-NMR (150 MHz, D₂O) : δ = 14.4 (C-12^{I}), 15.5 (CH₃-4^{F}), 17.2 (CH₃-C⁶), 23.0 (NHCOCH₃^{E}), 23.1 (NHCOCH₃^{C}), 23.2 (CH₂-11^{I}), 29.6-30.4 (CH₂-3^{I}-19^{I}), 32.4 (CH₂-10^{I}), 35.5 (CH₂-1^{I}), 46.5 (CH₂-NH^{I}), 55.8 (C-2^{E}), 55.9 (C-2^{C}), 61.2 (C-6^{D}), 69.3 (C-6^{E}), 70.2 (C-4^{B}), 71.0 (C-4^{D}), 71.5 (C-2^{B}), 72.5 (C-5^{C}), 72.6 (C-3^{E}), 72.8 (C-3^{C}), 73.2 (C-5^{F}), 73.5 (C-4^{F}), 73.6 (C-2^{D}), 73.9 (C-5^{B}), 74.6 (C-3^{F}), 75.3 (C-3^{D}), 76.2 (C-5^{D}), 76.4 (C-2^{F}), 80.0 (C-4^{E}), 83.5 (C-4^{C}), 94.7 (C-1^{F}), 101.7 (C-1^{C}), 102.4 (C-1^{E}), 103.0 (C-1^{B}), 103.7 (C-1^{D}), 117.5 (C-2^{I}, C-6^{I}), 129.5 (C-3'^{I}, C-5'^{I}), 135.6 (C-4'^{I}), 143.9 (C-1'^{I}), 158.6 (C-3^{F}), 173.4 (C-5^{B}), 173.6 (C-5^{C}), 174.7 (C-2^{E}), 175.0 (C-2^{C})

### Example 6 : 3-carbamate 1-[(2R)-2-carboxy-2-[(3-ethenyl-2-hydroxydodecyl)oxy]ethyl hydrogen phosphate] 0-.beta.-D-glucopyranosyl-(1.fwdarw.6)-O-[O-(.beta.-D-galactopyranuronamidosyl)-(1.fwdarw.4)-2-(acetylamino)-2,6-dideowy-.beta.-D-glucopyranosyl-(1.fwdarw.4]-O-2-(acetylamino)-2-deoxy-.beat.-D-glucopyranosyl-(1.fwdarw.2)-4-C-methyl-.alpha.-D-Glucopyranuronamide

### Synthesis of the E/Z-dodecenylbromide derivative (39) of the aldehyde (P)

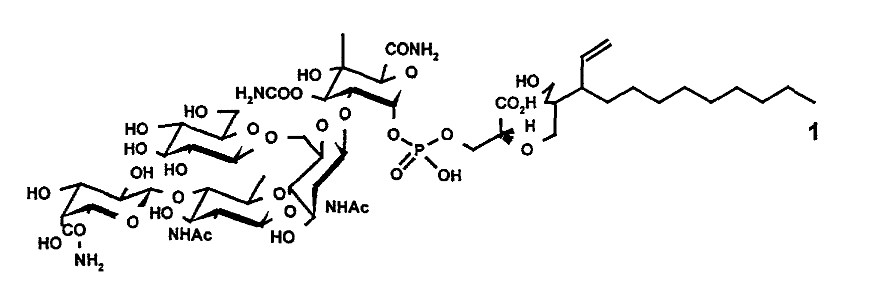

To a solution of product P (57 mg, 48 µmol) in a mixture of 400 µl of water and 1.6 ml of methanol 59.3 mg (240 µmol) E/Z-dodecenylbromide and 11 mg (96 µmol) indium were added. To this solution ultra sonic was applied for 10 min and rapid stirring for 24 h. The crude mixture was filtered and the filtrate was directly applied on a Sephadex® LH-20 column (10 g/ H₂O-CH₃OH 1 :4). All fractions containing the product were collected, evaporated and freeze dried. The crude product was purified on an silica gel column using AcOEt/ i-PrOH/H₂O (6 :4 :3) as eluent followed by another Sephadex LH-20 column to remove traces of silica gel. After solvent evaporation and lyophilisation the product **39** was obtained in 11 % (6.5 mg) yield.
C₅₃H₉₀N₅O₃₃P 1356.30 g/mol
MS: m/z (electron spray - positive mode)
1357.0 ([M+H]⁺), 1378.4 ([M+Na]⁺), 691.0 ([M+Na]²⁺), 679.1 ([M+2H]²⁺)
¹H-NMR δ = 0.9 (t, CH₃-1^{I}) 1.20 (s, CH₃-4^{F}), 1. 25 (s, CH₂-2-7^{I}), 1.38 (d, CH₃-6^{C}) , 2.00 (s,NHCOCH₃^{E}), 2.07 (s, NHCOCH₃^{C}), 5.4-5.6 (m, -CH=CH₂), 5.8 (q, H-1^{F})

### Example 7 : isomere Z corresponding to example 4

### Synthesis of the [¹⁵N]-Z-phytolamine derivative of the aldehyde (P)

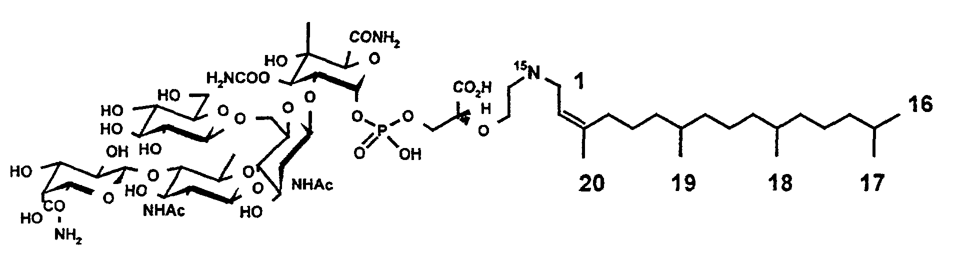

To a solution of **3** (65.2 mg, 55 µmol) in a mixture of 300 µl water, 100 µl phosphate buffer (pH 7, 0.2 M) and 800 µl methanol 48.8 mg (165 µmol) of [¹⁵N]-Z-phytolamine solved in 800 µl methanol and 6.9 mg (110 µmol) NaBH₃CN solved in 100 µl methanol were added. The pH of the mixture was adjusted with 1 N acetic acid to 7. The mixture was stirred for 48 h at room temperature. The crude mixture was directly applied on a Sephadex® LH-20 column (10 g/ H₂O-CH₃OH 1 :4). All fractions containing the product were collected, evaporated and freeze dried. The crude product was purified on an silica gel column using AcOEt/ i-PrOH/H₂O (6 :4 :3) as eluent followed by another Sephadex LH-20 column to remove traces of silica gel. The residue was taken up and freed from a second equivalent of [¹⁵N]-Z-phytolamine using a DOWEX® (type : 50 W X8) column (water). After solvent evaporation and lyophilisation the product **13** was obtained in 8 % ( 6.7 mg) yield.
C₆₁H₁₀₇N₆O₃₂P 1468.53 g/mol
MS: m/z (electron spray - positive mode)
1508.6 ([M+K]⁺), 1489.4 ([M+Na]⁺), 754.1 ([M+2K]²⁺)
¹H-NMR (600 MHz, CD₃OD): δ = 0.80-0.82 (s, CH₃- 16,17^{I}), 0.83 (s, CH₃- 18,19^{I}), 1.17 (s, CH₃-4^{F}), 1. 3 (CH₂-I), 1.35 (d, CH₃-6^{C}), 1.71 (s, CH₃-20^{I}) 1.94 (s,NHCOCH₃^{E}), 2.00 (s, NHCOCH₃^{C}), 2.09 (t, C**H**₂-CH=CH-) , 5.06 (d, 3-H^{F}), 5.36 (-C**H**=C**H**^{**I**}),5.97 (q, H-1^{F})

So, using the different processes, the following products were prepared

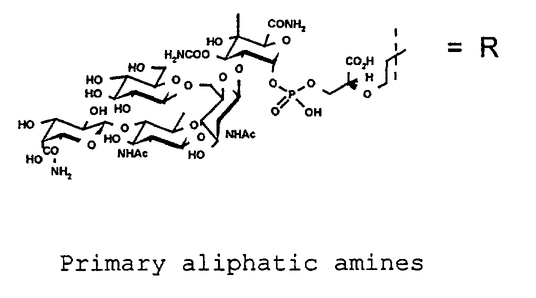

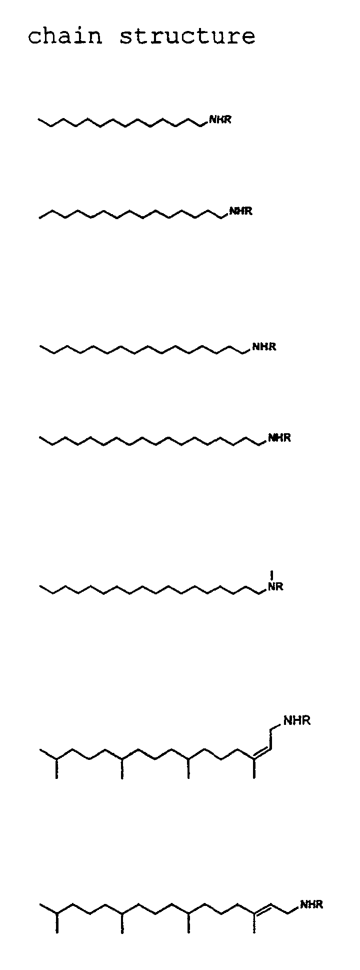

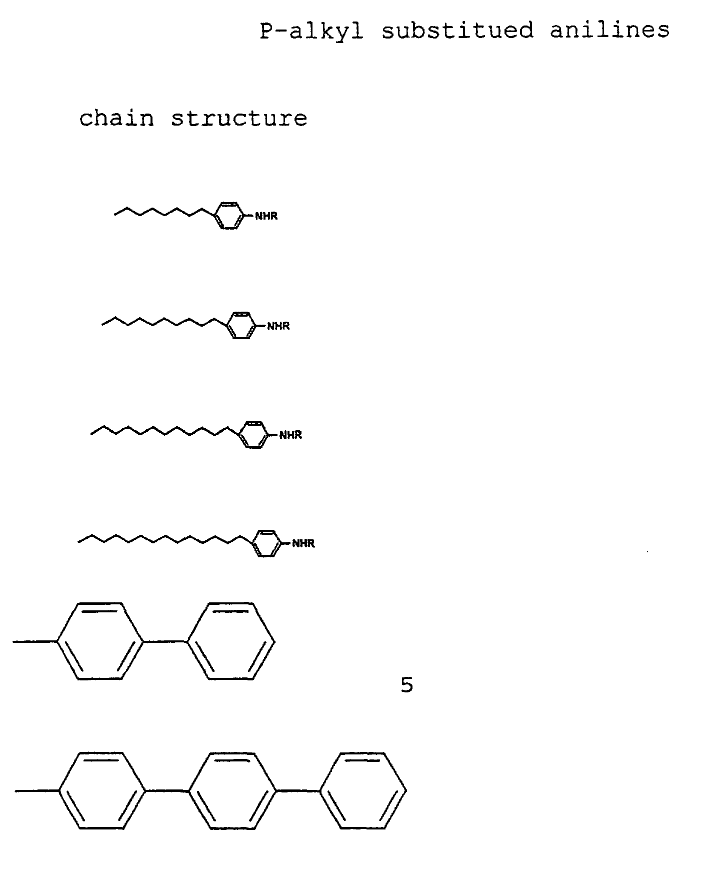

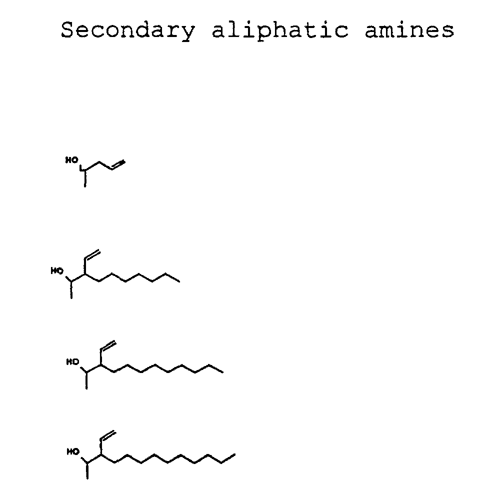

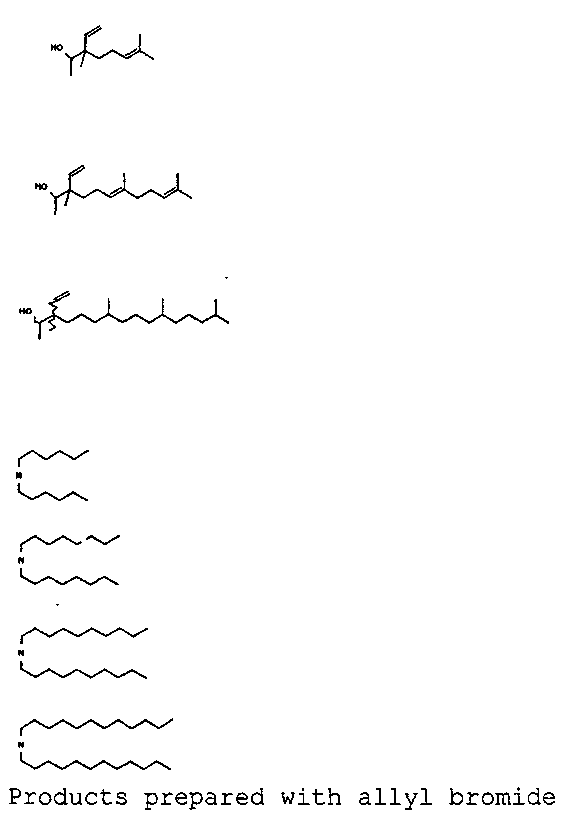

Example : Tablets were prepared containing 150 mg of the product of Example 4 or 5 or 6 and sufficient excipient of starch, talc and magnesium stearate for a final weight of 1 g.

### PHARMACOLOGICAL STUDY OF THE PRODUCTS OF THE INVENTION

### A) Activity in vitro

### Method of dilutions in liquid medium.

A series of tubes were prepared in which the same amount of sterile nutritive medium was distributed and increasing amounts of the product to be studied was distributed in each tube. Then, each tube was seeded with a bacterial strain and after incubationg for twenty-four hours in an oven at 37° C, the inhibition of growth was determined by transillumination which allowed the minimum inhibiting concentrations (M.I.C.) to be evaluated.

Products were tested on many strains inclusing :
S. aureus SG511
S. aureus SG511 (+10 % serum)
S. aureus Exp54146
S. pyogenes A561
E. faecium M78L

Good results were observed specially products of example 4, 5, 6 efficient against S. pyogenes A561 at dose ≤ 1,2 microgramme/ml.

## Claims

1. Compounds of formula 1 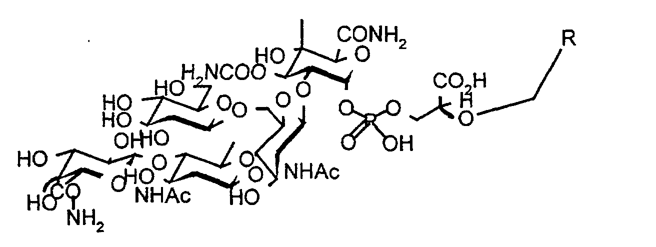 wherein
R is a radical 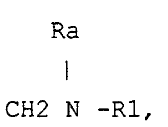 Ra being an hydrogen atom or an alkyl radical up to 8 carbon atoms, R1 being an hydrogen atom, an optionally substituted alkyl radical linear or branched up to 30 carbon atoms, saturated or unsaturated or an optionally substituted aryl radical up to 14 carbon atoms, or R is a radical 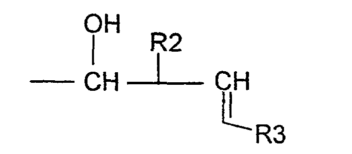 R2 being an hydrogen atom or an alkyl radical linear or branched, saturated or unsaturated, up to 30 carbonatoms R3 being an hydrogen atom or an alkyl radical up to 12 carbon atoms.

2. Compounds of formula 1 according to claim 1 wherein R is a radical 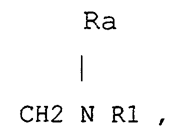 Ra and R1 being defined as above.

3. Compounds according to claim 2 wherein Ra is an hydrogen atom

4. Compounds according to claim 2 wherein R1 is aryl radical substituted by an alkyl radical in position para or a radical

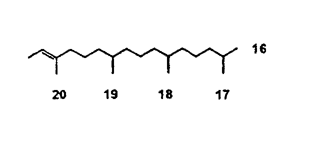

5. Compounds according to claim 1 wherein R is a radical 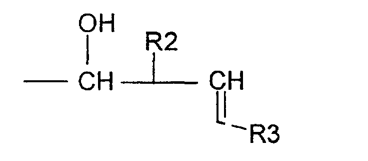 R2 and R3 being defined as above.

6. Compounds according to claim 5 wherein R2 is an alkyl radical comprising between 7 and 18 carbon atoms.

7. The following compounds according to claim 1 :
3-carbamate 1-[(2R)-2-carboxy-2-[[(3,7,11,15-tetramethyl-2-hexadecenyl)amino]ethoxy]ethyl hydrogen phosphate] 0-.beta.-D-glucopyranosyl-(1.fwdarw.6)-O-[O-(.beta.-D-galactopyranuronamidosyl)-(1.fwdarw.4)-2-(acetylamino)-2,6-dideoxy-.beta.-D-glucopyranosyl-(1.fwdarw.4]-O-2-(acetylamino)-2-deoxy-.beta.-D-glucopyranosyl-(1.fwdarw.2)-4-C-methyl-.alpha.-D-Glucopyranuronamide3-carbamate 1-[(2R)-2-carboxy-2-[[(4-dodecylphenyl))amino]ethoxy]ethyl hydrogen phosphate] 0-.beta.-D-glucopyranosyl-(1.fwdarw.6)-O-[O-(.beta.-D-galactopyranuronamidosyl)-(1.fwdarw.4)-2-(acetylamino)-2,6-dideoxy-.beta.-D-glucopyranosyl-(1.fwdarw.4]-O-2-(acetylamino)-2-deoxy-.beta.-D-glucopyranosyl-(1.fwdarw.2)-4-C-methyl-.alpha.-D-Glucopyranuronamide 3-carbamate 1-[(2R)-2-carboxy-2-[(3-ethenyl-2-hydroxydodecyl)oxy]ethyl hydrogen phosphate] 0-.beta.-D-glucopyranosyl-(1.fwdarw.6)-O-[O-(.beta.-D-galactopyranuronamidosyl)-(1.fwdarw.4)-2-(acetylamino)-2,6-dideowy-.beta.-D-glucopyranosyl-(1.fwdarw.4]-O-2-(acetylamino)-2-deoxy-.beat.-D-glucopyranosyl-(1.fwdarw.2)-4-C-methyl-.alpha.-D-Glucopyranuronamide

8. Process of preparation of compounds according to claim 1 comprising reacting moenomycin A to an ozonolysis reaction, obtaining the compound of formula II 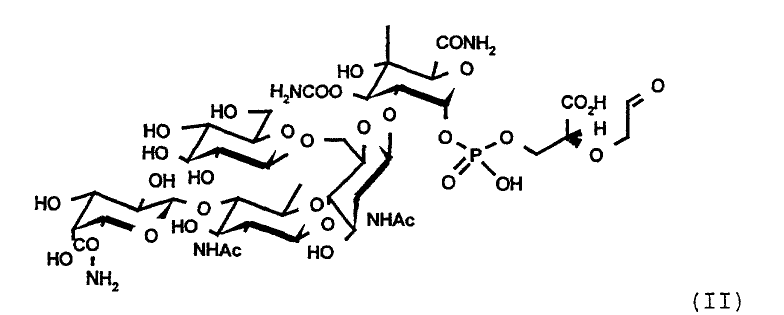 then submitting the compound of formula II to the action of a reagent or several reagents capable of remplacing 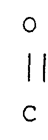 by the radical CHR to obtain the corresponding compound of formula I.

9. Process according to claim 8 wherein the compound of formula II is reacted with an amine of formula III 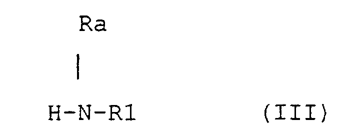 R a and Rb being defined as above,in the presence of reducing agent to obtain the corresponding compound of formula I.

10. Process according to claim 9, wherein the reducing agent is NaBH3CN.

11. Process according to claim 8 wherein a compound of formula II is submitted to an allylhalogenide to get the compound of formula I wherein R is a radical 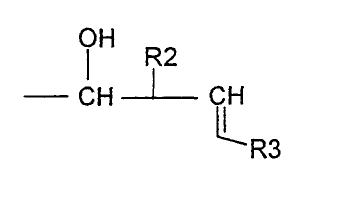 R2 and R3 being defined as above.

12. As a chemical product ,the compound of formula II as defined in claim 8.

13. As antibacterial products, the compounds of any of the claims 1 to 7.

14. Feed additives including as antibacterial products at least one compound of formula I as defined in any of the claims 1 to 7.

## Patentansprüche

1. Verbindungen der Formel I 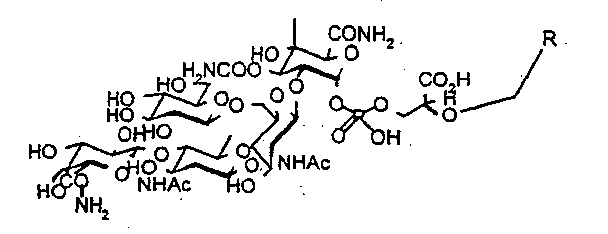 worin
R für eine Gruppe 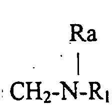 steht, worin
Ra für ein Wasserstoffatom oder eine Alkylgruppe mit bis zu 8-Kohlenstoffatomen steht, R₁ für ein Wasserstoffatom, eine gegebenenfalls substituierte, lineare oder verzweigte, gesättigte oder ungesättigte Alkylgruppe mit bis zu 30 Kohlenstoffatomen oder eine gegebenenfalls substituierte Arylgruppe mit bis zu 14 Kohlenstoffatomen steht, oder R für eine Gruppe 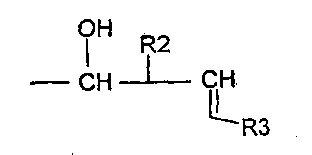 steht, worin R₂ ein Wasserstoffatom oder eine lineare oder verzweigte, gesättigte oder ungesättigte Alkylgruppe mit bis zu 30 Kohlenstoffatomen und R₃ ein Wasserstoffatom oder eine Alkylgruppe mit bis zu 12 Kohlenstoffatomen ist.

2. Verbindungen der Formel 1 nach Anspruch 1, worin
R für eine Gruppe 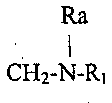 steht und Ra und R₁ wie oben definiert sind.

3. Verbindungen nach Anspruch 2, worin Ra ein Wasserstoffatom ist.

4. Verbindungen nach Anspruch 2, worin R₁ eine in para-Position durch eine Alkylgruppe substituierte Arylgruppe oder eine Gruppe 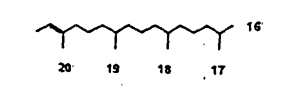 ist.

5. Verbindungen nach Anspruch 1, worin R eine Gruppe 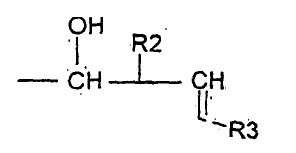 ist, wobei R₂ und R₃ wie zuvor definiert sind.

6. Verbindungen nach Anspruch 5, worin R₂ eine Alkylgruppe mit zwischen 7 und 18 Kohlenstoffatomen ist.

7. Nachstehende Verbindungen gemäß Anspruch 1:
3-Carbamat-1-[(2R)-2-carboxy-2-[[(3,7,11,15-tetramethyl-2-hexadecenyl)-amino]ethoxy]ethylhydrogenphosphat]-0-β-D-glucopyranosyl-(1→ 6)-O-[O-β-D-galactopyranuronamidosyl)-(1→ 4)-2-(acetylamino)-2,6-dideoxy-β-D-glucopyranosyl-(1→ 4]-O-2-(acetylamino)-2-deoxy-β-D-glucopyranosyl-(1→ 2)-4-C-methyl-α-D-glucopyranuronamid,
3-Carbamat-1-[(2R)-2-carboxy-2-[((4-dodecylphenyl))amino]ethoxy]ethylhydrogenphosphat]-O-β-D-glucopyranosyl-(1→ 6)-O-[O-β-D-galactopyranuronsamidosyl)-(1→ 4)-2-(acetylamino)-2,6-dideoxy-β-D-glucopyranosyl-(1→ 4]-O-2-(acetylamino)-2-deoxy-β-D-glucopyranosyl-(1→ 2)-4-C-methyl-α-D-glucopyranuronamid,
3-Carbamat-1-[(2R)-2-carboxy-2-[(3-ethenyl-2-hydroxydodecyl)oxy]ethylhydrogenphosphat]-O-β-D-glucopyranosyl-(1→ 6)-O-[O-(β-D-galactopyranuronamidosyl)-(1→ 4)-2-(acetylamino)-2,6-dideoxy-β-D-glucopyranosyl-(1→ 4]-O-2-(acetylamino)-2-deoxy-β-D-glucopyranosyl-(1→ 2)-4-C-methyl-α-D-Glucopyranuronamid.

8. Verfahren zur Herstellung von Verbindungen nach Anspruch 1, wobei Moenomycin A in einer Ozonolyse zu einer Verbindung der Formel II umgesetzt wird 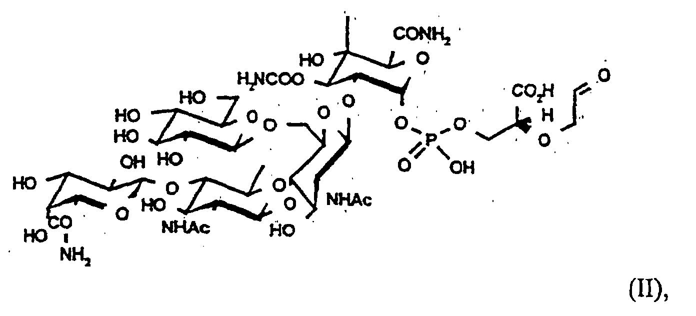 und die Verbindung der Formel II anschließend gegenüber der Wirkung eines oder mehrerer Reagenzien, die fähig sind, 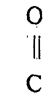 durch die Gruppe CHR zu ersetzen, ausgesetzt wird, so dass die entsprechende Verbindung der Formel I entsteht.

9. Verfahren nach Anspruch 8, worin die Verbindung der Formel II in Gegenwart eines Reduktionsmittels mit einem Amin der Formel III umgesetzt wird 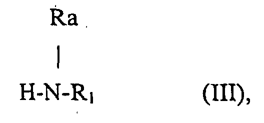 worin Ra und Rb wie oben definiert sind, so dass die entsprechende Verbindung der Formel I entsteht.

10. Verfahren nach Anspruch 9, worin das Reduktionsmittel NaBH₃CN ist.

11. Verfahren nach Anspruch 8, worin eine Verbindung der Formel II einem Allylhalogenid ausgesetzt wird, so dass die Verbindung der Formel I entsteht, worin R eine Gruppe 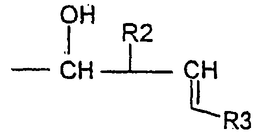 ist und R₂ und R₃ wie oben definiert sind.

12. Als chemisches Produkt die wie in Anspruch 8 definierte Verbindung der Formel II.

13. Als antibakterielle Produkte die Verbindungen einer der Ansprüche 1 bis 7.

14. Nahrungsmitteladditive, welche als antibakterielle Produkte wenigstens eine wie in einem der Ansprüche 1 bis 7 definierte Verbindung der Formel I enthalten.

## Revendications

1. Composés de formule 1 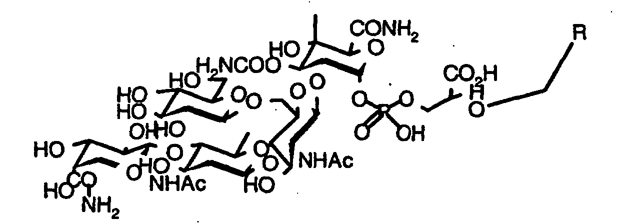 dans laquelle
R représente un radical 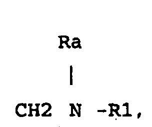 Ra étant un atome d'hydrogène ou un radical alkyle ayant jusqu'à 8 atomes de carbone, R1 étant un atome d'hydrogène, un radical alkyle éventuellement substitué, linéaire ou ramifié, saturé ou insaturé, ayant jusqu'à 30 atomes de carbone, ou un radical aryle éventuellement substitué, ayant jusqu'à 14 atomes de carbone,
ou R représente un radical 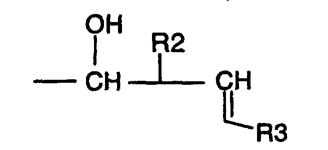 R2 étant un atome d'hydrogène ou un radical alkyle linéaire ou ramifié, saturé ou insaturé, ayant jusqu'à 30 atomes de carbone, R3 étant un atome d'hydrogène ou un radical alkyle ayant jusqu'à 12 atomes de carbone.

2. Composés de formule 1 selon la revendication 1, dans laquelle R représente un radical 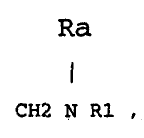 Ra et R1 étant tels que définis ci-dessus.

3. Composés selon la revendication 2, dans lesquels Ra représente un atome d'hydrogène.

4. Composés selon la revendication 2, dans lesquels R1 représente un radical aryle substitué par un radical alkyle en position para ou un radical

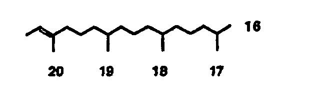

5. Composés selon la revendication 1, dans lesquels R représente un radical 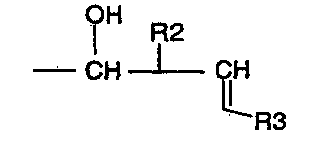 R2 et R3 étant tels que définis ci-dessus.

6. Composés selon la revendication 5, dans lesquels R2 représente un radical alkyle ayant entre 7 et 18 atomes de carbone.

7. Composés selon la revendication 1, comme suit :
le 3-carbamate de 1-[phosphate acide de (2R)-2-carboxy-2-[[(3,7,11,15-tétraméthyl-2-hexadécényl)amino]éthoxy]éthyl]-O-β-D-glucopyranosyl-(1 → 6)-O-[O-(β-D-galactopyranuronamidosyl)-(1 **→** 4)-2-(acétylamino)-2,6-didésoxy-β-D-glucopyranosyl-(1 → 4)-O-2-(acétylamino)-2-désoxy-β-D-glucopyranosyl-(1 → 2)-4-C-méthyl-α-D-glucopyranuronamide
le 3-carbamate de 1-[phosphate acide de (2R)-2-carboxy-2-[[(4-dodécylphényl))amino]éthoxy]éthyl]-0-β-D-glucopyranosyl-(1 → 6)-O-[O-(β-D-galactopyranuronamidosyl)-(1 → 4)-2-(acétylamino)-2,6-didésoxy-β-D-glucopyranosyl-(1 → 4)-O-2-(acétylamino)-2-désoxy-β-D-glucopyranosyl-(1 → 2)-4-C-méthyl-α-D-glucopyranuronamide
le 3-carbamate de 1-[phosphate acide de (2R)-2-carboxy-2-[(3-éthényl-2-hydroxydodécyl)oxy]éthyl]-0-β-D-glucopyranosyl-(1 → 6)-O-[O-(β-D-galactopyranuronamidosyl)-(1 → 4)-2-(acétylamino)-2,6-didésoxy-β-D-glucopyranosyl-(1 → 4)-O-2-(acétylamino)-2-désoxy-β-D-glucopyranosyl-(1 → 2)-4-C-méthyl-α-D-glucopyranuronamide.

8. Procédé de préparation de composés selon la revendication 1, comprenant la soumission de moénomycine A à une réaction d'ozonolyse, pour obtenir le composé de formule II 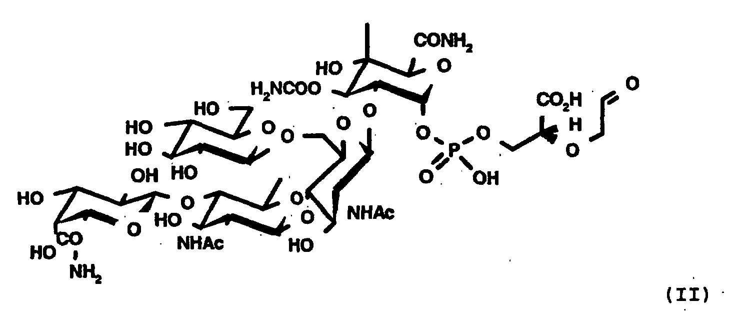 puis la soumission du composé de formule II à l'action d'un ou plusieurs réactif(s) capable(s) de remplacer 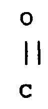 par le radical CHR pour obtenir le composé correspondant de formule I.

9. Procédé selon la revendication 8, dans lequel le composé de formule II est réagi avec une amine de formule III 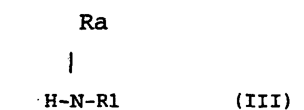 Ra et Rb étant tels que définis ci-dessus, en présence d'un agent réducteur pour obtenir le composé correspondant de formule I.

10. Procédé selon la revendication 9, dans lequel l'agent réducteur est NaBH₃CN.

11. Procédé selon la revendication 8, dans lequel un composé de formule II est soumis à un halogénure d'allyle pour obtenir le composé de formule I, dans laquelle R est un radical 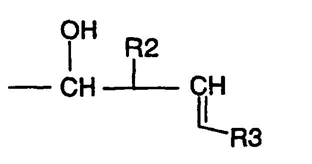 R2 et R3 étant tels que définis ci-dessus.

12. Composé de formule II tel que défini selon la revendication 8, comme produit chimique.

13. Composés selon l'une quelconque des revendications 1 à 7, comme produits antibactériens.

14. Additifs alimentaires comportant, comme produit antibactérien, au moins un composé de formule I tel que défini selon l'une quelconque des revendications 1 à 7.
